(19) Europäisches Patentamt
European Patent Office
Office européen des brevets

(11) Publication number: **0 073 089**
**B1**

(12) # EUROPEAN PATENT SPECIFICATION

(45) Date of publication of patent specification: **12.06.85**

(51) Int. Cl.⁴: **C 12 Q 1/16,** B 01 D 15/04

(21) Application number: **82300828.9**

(22) Date of filing: **18.02.82**

(54) **Culture medium and method for culturing body fluids containing antimicrobials.**

(30) Priority: **20.08.81 US 294857**

(43) Date of publication of application:
**02.03.83 Bulletin 83/09**

(45) Publication of the grant of the patent:
**12.06.85 Bulletin 85/24**

(84) Designated Contracting States:
**BE DE FR GB IT NL SE**

(56) References cited:
**GB-A-1 441 022**
**US-A-3 794 584**
**US-A-4 145 304**
**US-A-4 174 277**

**APPLIED MICROBIOLOGY, vol 22, no. 5, November 1971, H.J. DEBLANC et al. "Automated radiometric detection of bacteria in 2,967 blood cultures", pages 846 to 849**

(73) Proprietor: **Becton, Dickinson and Company**
**Mack Centre Drive**
**Paramus New Jersey 07652 (US)**

(72) Inventor: **Broman, Rodney L.**
**2127, Buell Drive**
**Fallston Maryland (US)**
Inventor: **Waters, John R.**
**801, Stags Head Road**
**Towson Maryland (US)**

(74) Representative: **Ruffles, Graham Keith et al**
**MARKS & CLERK 57-60 Lincoln's Inn Fields**
**London WC2A 3LS (GB)**

**EP 0 073 089 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This present invention is directed generally to an improved means for the detection of biological activity in a body fluid sample containing an antibiotic or other antimicrobial inhibitor. More particularly, the present invention is directed to an improved microbial growth medium containing an effective amount of a substance capable of isolating antimicrobial inhibitors.

Background of the invention

Biological activity in blood, such as bacteremia (bacteria in blood), remains a significant problem despite the availability of antimicrobial drugs. In particular, the rapid isolation of offending organisms from bacteremic patients is made more difficult when the patient has been administered antibiotics, which are transferred along with the bacteria in the blood into culture broths and often inhibit the growth of the organism. It is nevertheless important that the identification and susceptibility of an infecting organism to various antimicrobial drugs be determined as early as possible in the course of bacteremia.

When conventional culturing techniques are employed to identify an infecting organism, the administration of an antibiotic prior to drawing the blood sample for testing can result in inhibition of the growth of bacteria, thus interferring with isolation and identification of the offending bacterium. Even where antibiotics may not be present in the blood, the isolation of the offending organism(s) still may require excess periods of incubation because of inhibitors contained in serum, plasma, or lysed erythrocytes.

Similar problems exist despite the type of body fluid being examined, whether it is urine, spinal fluid, abscess exudates, serum, peritoneal fluid and the like.

The usual method for detection of bacteria is to inoculate 5 ml of a body fluid into a culture medium and wait for the appearance of turbidity which is an indication of bacterial growth. Patients who have been subjected to antibiotic therapy will have the antibiotic present in the body fluid at the time the fermentation is initiated. Presence of the antibiotic inhibits growth of the bacteria and may delay isolation of the bacteria for as long as 14 days.

More recently, a radiometric technique for the detection of biological activity in the blood has undergone clinical testing and has been adopted for commercial practice. In that method, samples of blood are inoculated into a suitable growth medium that includes a $C^{14}$ containing carbon source, the inoculated medium is incubated for a suitable period, and a portion of the gaseous atmosphere is analyzed for $C^{14}O_2$ while in the gaseous state. Such process is described, inter alia, in U.S. Pat. No. 3,676,679 issued July 11, 1972; and in the articles "Early Detection of Bacterial Growth, with Carbon$^{14}$ Labeled Glucose," Radiology, 92, No. 1, pp. 154—5 (Jan. 1969); "Automated Radiometric Detection of Bacterial Growth in Blood Culture," J. Labs. Clin. Med., 75, No. 3, pp. 529—34 (March 1970); and "Automated Radiometric Detection of Bacteria in 2,967 Blood Cultures," Applied Microbiology, 22, No. 5, pp. 846—849 (Nov. 1979). A commercial instrument for the practice of a rapid, automated process is available under the trademark BACTEC (Johnston Laboratories). Although this method can be used to rapidly determine the presence of bacteria in a culture in the absence of antibiotics or their inhibitors, it is inefficient when growth is attempted in the presence of antibiotics or other inhibitors in the culture media.

Antibiotics can be separated from micro-organisms by membrane chromatography, but these procedures are not practical because of the complexity of the separation technique and the high rate of contamination of the test culture.

United States Patent Number 4,174,277 to Melnick et al. discloses a method for the separation of antibiotics from bacteria in a body fluid sample. In the method of the Melnick patent, an antibiotic is selectively removed from a bacterially infected body fluid specimen by adsorbing the antibiotic onto a resin system treated with a detergent. The detergent renders the resin system selective to the antibiotic while permitting the bacteria to remain free in the eluting fluid and thus the bacteria are separated from the antibiotic. The eluted body fluid specimen containing the bacteria is then inoculated into a growth medium and cultured.

While the method of the Melnick et al. patent has been useful in overcoming the problem of antibiotic inhibitor contamination of blood samples, it introduces a separate handling step in the treatment of body fluid samples prior to the conventional culturing process. The separate handling step is particularly cumbersome and may introduce contamination when handling a multitude of samples as is done with current automatic fermentation apparatus.

It is therefore, a principal object of the present invention to provide a culture medium for the growth and detection of an infecting organism in a body fluid specimen without the need to resort to a separate step for separation of an antibiotic or inhibiting substance.

It is another object of this invention to provide a means for isolating antimicrobial inhibitors from body fluid specimens during growth of an organism in culture medium.

It is a further object of the present invention to provide a means for isolating antibiotics from an infected body fluid specimen without significantly affecting the metabolism of the micro-organism population of the specimen during growth in culture medium.

It is yet another object of the present invention to provide a method utilizing a resin which will isolate

antibiotics and other inhibitors contained in a body fluid specimen while exhibiting little effect on growth of microorganisms in the specimen.

It is still another object of the present invention to provide a resin for isolating materials inhibitory to microorganisms in a body fluid specimen without affecting the microbial population of the specimen.

Summary of the invention

This invention relates to a culture medium for growth of microorganisms containing an effective amount of a substance capable of isolating antimicrobial materials in an infected body fluid specimen. The isolating substances of the invention are ion exchange resins and nonionic adsorbent resins, characterized by their capacity for isolation of antibiotics and other inhibitors.

Although ion exchange resins and adsorbent resins are known to adsorb charged antibiotics from fluid specimens, they have not proven to be a satisfactory means for removing antibiotics from bacterially infected specimens where rapid isolation and identification of an infecting bacterium is the objective. The reason attributed to this lack of success in the use of ion exchange resins and adsorbent resins for treatment of antibiotic containing specimens is that upon passage of a specimen containing an antibiotic and an infecting organism through a resin, the resultant filtrate is not only substantially freed of the antibiotic, but the infection bacteria are also significantly removed by the resin.

For the foregoing reasons many research efforts have been directed to modifying ion exchange resins and/or nonionic adsorbent resins to provide a resin capable of removing the antibiotic or other inhibitor without removal of the infecting bacteria from the body fluid sample. U.S. Patent Number 4,174,277 to Melnick et al. is exemplary of these efforts to modify resins for selective removal of antibiotics and other bacterial inhibitors from the body fluid sample.

Because of the effectiveness of ionic exchange resins and nonionic adsorbent resins for removal of infecting microorganisms, as well as nutrients and antibiotics, from a culture medium, it has been thought by the industry to be impossible to isolate antibiotics and other inhibitors from the nutrient broth without, at the same time, isolating the microorganism and the nutrients contained in the culture medium including a body fluid sample.

In accordance with the present invention, it has now been discovered that ion exchange resins and nonionic adsorbent resins can be present in a culture medium during culturing of a body fluid sample without interference with the ability of the infecting microorganism to grow in the culture medium while at the same time effectively isolating the antibiotic or other inhibitor from interference with microorganism growth. Rapid culturing and analysis of the microorganism in the specimen are thereby made possible without a separate separation step to remove antibiotics and other inhibitors.

Detailed description of the invention

In accordance with the present invention a substance capable of isolating an antimicrobial is added to a culture medium to provide a stabilized culture medium. The stabilized culture medium can then be used to culture a body fluid specimen infected with a microorganism and contaminated with an antibiotic or other antimicrobial while isolating the antimicrobic from interfering with growth of the microorganism contained in the specimen.

The substances which may be employed in the present invention to isolate antimicrobics in culture media are resins or combinations of resins which are known to adsorb antimicrobics. It is to be understood that throughout this application the adsorption by the resins of the invention refers to the ability of the resins to isolate microorganism growth inhibitors without interference with microorganism growth. The physical effect of the resins on other materials which may be present in the culture medium, including the infecting microrganism, is not significant so long as the microorganism growth inhibitor is isolated from interference with microorganism growth.

More particularly, the resins which may be used in the practice of the present invention include ion exchange resins and non-functional polymeric resin adsorbents. The resins which may be employed in the practice of the invention include synthetic ion exchange resins and non-functional nonionic adsorbent resins with matrices formed from condensation and addition polymers. Specifically, polystyrene resins cross-linked with divinyl benzene may be used.

Sodium, hydrogen and ammonium charged cation exchange resins have been found to be particularly suitable for use in the present invention. The following cation exchange resins have been found to be effective:

BIO REX
AG 50W—$X_2$, $X_4$, $X_6$, $X_8$, $X_{10}$, $X_{12}$, and $X_{16}$

from BIO-RAD Laboratories,

DOWEX
50W—$X_2$, $X_4$, $X_8$, $X_{10}$, $X_{12}$ and $X_{16}$

from Dow Chemical Company and Rexyn 101 from Fisher Scientific Co., all of which are strong acid polystyrene resins having $SO_3^-$ functional group. The cation exchange resins are particularly useful in

combination with the non-functional resins, such as the XAD resins manufactured by Rohm & Haas and SM resins manufactured by BioRad.

Chloride, formate, acetate and hydroxide, charged anion exchange resins have generally been found to be suitable. Specifically, chloride charged anionic exchange resins in combination with adsorbent resins sold under the following trademarks have been found effective in the practice of the invention: DOWEX 1—X8 from Dow Chemical Company, DUOLITE A-109 from Diamond Shamrock Company and AMBERLITE IRA400 from Rohm & Haas, all of which are strong base resins having polystyrene quaternary ammonium functional groups; DUOLITE A-7 from Diamond Shamrock Company and AMBERLITE IR45 from Rohm & Haas, which are weakly basic and have tertiary amine functional groups. The anion exchange resins are preferably used in combination with a non-functional resin such as the XAD resins from Rohm & Haas and SM resin from BioRad, particularly XAD-4 resin which is a non-functional copolymer of styrene and divinyl benzene.

It should be understood that the pore size of the resin is not critical or important in the practice of the present invention. Generally, resins do not have a pore size sufficient to permit bacteria to penetrate into the interior of the resin. Certain resins, however, have macroporous structures with large internal surfaces which permit large molecules to penetrate their interiors. Such macroporous resins are entirely suitable for use in the practice of the present invention since entrapment of the bacteria within the pores does not necessarily limit growth of the bacteria in the presence of the culture medium. Resins which have relatively smaller pores wherein adsorption is effected principally on the external surfaces of the resin, that is, microporous resins are also suitable in the practice of the invention. A particularly preferred combination is a non-functional polymeric adsorbent resin, such as XAD-4 and a cationic exchange resin. Such a combination resin isolates antibiotics as well as other bacterial inhibitors, while not interfering significantly with the growth of microorganisms contained in a body fluid specimen.

In general, it is preferred to use from about 0.5 to about 5 grams (dry weight basis) of a non-functional polymer adsorbent resin in combination with from about 0.05 to about 1.0 grams (dry weight basis) of an ion exchange resin for each 30 ml of microbial growth media. At the above indicated levels of resin, the growth medium is suitable for culturing up to about 10 ml of body fluid specimen, preferably from about 2 ml to about 5 ml of body fluid specimen.

The resins of the invention will isolate antimicrobials and other inhibitors from infected body fluid specimens including urine, blood, spinal fluid and the like. Generally, the body fluid specimen is transferred aseptically to a vial containing the resins and a suitable culture medium by perforating the septum of the vial with the syringe needle and injecting a sample of the body fluid specimen into the vial. The vial is placed on a shaker or other suitable device and shaken for a sufficient time to allow contact of the resin and the specimen. Any other type of apparatus or method which provides sufficient contact between the resin and the specimen to assure contact of the antibiotic or other antimicrobic with the resin may also be employed.

After shaking, tumbling or otherwise contacting the specimen with the resin, the vial containing the resin, the culture medium and the specimen is maintained under suitable incubation conditions to permit fermentation of the microorganisms in the specimen. Shaking may continue during the culturing process.

Generally, the process employs a nutrient culture medium that contains water, a suitable $C^{14}$ containing carbon source, a nitrogen source minerals and trace elements. Typical $C^{14}$ containing carbon sources may be glucose, fructose, galactose, mannose, rhamnose, or the like, phenylalanine, lysine, arginine or the like, glycerol, urea, or carboxylic acids such as citric acid or the like. Glucose, which is readily available, constitutes a preferred $C^{14}$ containing material. Generally the levels of radioactivity will vary from about 0.1 to about 10 microcuries per 10 ml. The assimilable nitrogen source may be either organic or inorganic, such as nitrates, nitrites, ammonia, urea, amino acids, or the like, while minerals such as the chlorides, sulfates or phosphates of calcium, sodium, potassium, magnesium or the like of trace elements such as manganese, iron, zinc, cobalt or the like, may also be employed. Vitamins, cofactors or other enrichment agents such as anti-coagulants may also be added if desired. Finally, the medium may also include a buffer for pH adjustment and maintenance. The atmosphere above the culture medium can be air, oxygen, or the like if aerobic tests are being conducted, whereas nitrogen, $CO_2$, or the like can be employed if anaerobic tests are being conducted.

A broad selection of possible components that may be included in the media is also set out in U.S. Pat. No. 3,676,679. While that patent indicates that up to 20 percent or more carbohydrate may be employed in the medium, preferred media contain up to about 0.5 percent carbohydrate and commercial media do contain such small amounts of carbohydrate added as such. In addition, if the media contains peptone, yeast extract, or the like, an additional 1 percent or so of carbohydrates may be present as a component of this additive.

Vials containing media for aerobic cultures, designated No. 6, and vials containing media for anaerobic cultures, designated No. 7, are commercially available from Johnston Laboratories. The vials are of 50 ml nominal capacity and contain 30 ml of culture medium and have a radioactivity of about 2 microcuries. A medium suitable for aerobic fermentation (6) may contain tryptic soy broth, hemin, menadione, sodiumpolyanethol sulfonate, and $C^{14}$-labelled substrates, while a medium suitable for anaerobic culture (7) may contain tryptic soy broth, yeast extract, hemin, menadione, L-cysteine, sodiumpolyanethol

sulfonate and $C^{14}$-labelled substrates. The 50 ml vials contain 30 ml of medium and have a radioactivity of about 2 microcuries. Commercial media have a pH of about 7.3.

Where a specimen to be analyzed contains a known and identified antibiotic, a resin which is known to be effective to isolate that antibiotic is selected and added to the culture media containing the body fluid specimen. Where a specimen contains one or more unidentified antibiotics, a combination of resins which will isolate various antibiotics and permit growth of the microorganisms is used. The specimen is contacted with the selected resins in accordance with the above-described methods during fermentation.

The following examples further illustrate various features of the invention but are not intended to any way limit the scope of the invention which is defined in the appended claims.

Example

To determine whether resins had an effect on the ability of a medium to support growth, blood specimens were prepared by adding a particular microorganism at a level of 100 colony forming units (CFU) or less to 5 ml whole blood samples. The inoculated blood specimens were added to 500 ml vials containing either 30 ml of a culture medium or 30 ml of a culture medium containing 4 grams of XAD-4 nonionic polymeric resin adsorbent (Rohm & Haas) and 0.25 grams of a cationic exchange resin, sodium form, such as Rexyn 101 resin (Fisher Scientific). The culture medium, designated 6B, has the following formulation:

| List of ingredients | Amount |
|---|---|
| Purified Water | 30 ml |
| Tryptic Soy Broth | 2.75% W/V |
| Hemin | 0.0005% W/V |
| Vitamin K | 0.00005% W/V |
| $^{14}C$-Labelled Substrates | 2.0 μ Ci |
| Sodium Bicarbonate | 0.0375% W/V |
| $CO_2$ Atmosphere | 10% VV |
| Sucrose | 0.25% W/V |
| Pyridoxal HCL (Vitamin $B_6$) | 0.001% W/V |
| Sodiumpolyanethol Sulfonate | 0.025% W/V |

The separate vials containing blood specimens inoculated with particular microorganisms were placed in a reciprocating shaker and incubated for 24 hours at 37°C. Samples containing the particular microorganisms were grown in separate vials containing the culture medium and were also grown in separate vials containing the culture medium with the above described resins. The results of the growth comparisons are set forth hereinbelow in Table 1 (+ means that growth was detected).

TABLE 1

Growth in culture medium with resins

| | 6B | 6B+Resins |
|---|---|---|
| *S. aureus* | + | + |
| *E. coli* | + | + |
| GpD Streptococcus | + | + |
| *N. meningitidis* | + | + |
| *N. gonorrheae* | + | + |
| *P. aeruginosa* | + | + |
| *P. alcaligenes* | + | + |
| *P. testosterosis* | + | + |
| *S. bovis* | + | + |
| Streptococcus | + | + |
| *Moraxella sp* | + | + |
| *S. morbillorium* | + | + |
| *S. pneumoniae* | + | + |
| *P. acne* | + | + |
| *K. pneumoniae* | + | + |
| *H. influenzae* | + | + |
| *Candida albicans* | + | + |
| *Torulopsis glabrata* | + | + |

Thereafter, the 5 ml blood specimen and various organisms were inoculated into the culture media followed by addition of antibiotics as set forth hereinbelow in Table 2. Again, dual samples were fermented, one of the samples containing only the 6B culture medium and the other sample containing the 6B culture medium plus the above described resin. In each case, as shown hereinbelow in Table 2, the culture medium with the resin provided positive growth even in the presence of the antimicrobics.

TABLE 2
Protective effect of resins

| Organism | Drug (conc/vial) | Growth 6B | Growth 6B+Resin |
|---|---|---|---|
| *S. aureus* | Penicillin G (0) | + | + |
| | Penicillin G (100 units) | − | + |
| | Amikacin (0) | + | + |
| | Amikacin (200 μg) | − | + |
| | Erythromycin (0) | + | + |
| | Erythromycin (250 μg) | − | + |
| | Methicillin (0) | + | + |
| | Methicillin (250 μg) | − | + |
| *P. aeruginosa* | Amikacin (0) | + | + |
| | Amikacin (200 μg) | − | + |
| | Gentamicin (0) | + | + |
| | Gentamicin (140 μg) | − | + |
| *E. coli* | Gentamicin (0) | + | + |
| | Gentamicin (140 μg) | − | + |
| *Candida albicans* | Amphotericin B (0) | + | + |
| | Amphotericin B (100 μg) | − | + |
| *S. aureus* | Gentamicin (0) and Ampicillin (0) | + | + |
| | Gentamicin (140 μg) and Ampicillin (250 μg) | − | + |

**Claims**

1. A method for detecting biological activity in a body fluid sample which may contain antimicrobial material wherein a growth medium is inoculated with the body fluid sample, the inoculated growth is cultured and metabolic byproducts are monitored to detect biological activity, characterized in that a resin capable of isolating any antimicrobial material present in the body fluid sample without hindering biological activity during the culturing period is incorporated into said growth medium, the resin being an ion exchange resin and/or a non-functional resin adsorbent.

2. A method according to Claim 1, wherein the resin is an ion exchange resin.

3. A method according to Claim 1, wherein the resin is a non-functional resin adsorbent.

4. A method according to Claim 1, wherein the resin is a non-functional resin adsorbent together with an ion exchange resin.

5. A method according to Claim 2 or 4, wherein the ion exchange resin is a cationic exchange resin.

6. A method according to Claim 5, wherein the cationic exchange resin is in the sodium form.

7. A method according to Claim 1, wherein the resin is a non-functional copolymer of divinyl benzene and styrene, an ion exchange resin which is a copolymer of divinyl benzene and styrene, or a mixture thereof.

8. A method according to any preceding claim, wherein the body fluid sample is blood, urine, spinal fluid or peritoneal fluid.

9. A growth medium for detecting biological activity in a body fluid sample which may contain antimicrobial material, the medium comprising an aqueous dispersion of biological nutrient materials and an effective amount of a resin capable of isolating antimicrobial materials without hindering biological activity during culturing of a body fluid sample, the resin being an ion exchange resin and/or a non-functional resin adsorbent.

10. A growth medium according to Claim 9, wherein the resin is an ion exchange resin.

11. A growth medium according to Claim 9, wherein the resin is a non-functional resin adsorbent.

12. A growth medium according to Claim 9, wherein the resin is a non-functional resin adsorbent together with an ion exchange resin.

13. A growth medium according to Claim 10 or 12, wherein the ion exchange resin is a cationic exchange resin.

14. A growth medium according to Claim 13, wherein the cationic exchange resin is in the sodium form.

15. A growth medium according to Claim 9, wherein the resin is a non-functional copolymer of divinyl benzene and styrene, an ion exchange resin which is a copolymer of divinyl benzene and styrene, or a mixture thereof.

**Patentansprüche**

1. Verfahren zur Bestimmung biologischer Aktivität in einer Probe einer Körperflüssigkeit, die antimikrobielles Material enthalten kann, wobei ein Nährmedium mit der Probe der Körperflüssigkeit beimpft wird, das beimpfte Nährmedium kultiviert und Stoffwechsel-Nebenprodukte zur Bestimmung biologischer Aktivität kontrolliert werden, dadurch gekennzeichnet, daß ein Harz, das zur Isolierung jedweden antimikrobiellen Materials, das in der Probe der Körperflüssigkeit enthalten ist, fähig ist, ohne die biologische Aktivität während des Kultivierungszeitraums zu stören, dem Nährmedium einverleibt wird, und zwar ein Ionenaustauscherharz und/oder ein nicht-funktionelles Adsorberharz.

2. Verfahren nach Anspruch 1, dadurch gekennzeichnet, daß das Harz ein Ionenaustauscherharz ist.

3. Verfahren nach Anspruch 1, dadurch gekennzeichnet, daß das Harz ein nicht-funktionelles Adsorberharz ist.

4. Verfahren nach Anspruch 1, dadurch gekennzeichnet, daß das Harz ein nicht-funktionelles Adsorberharz zusammen mit einem Ionenaustauscherharz ist.

5. Verfahren nach Anspruch 2 oder 4, dadurch gekennzeichnet, daß das Ionenaustauscherharz ein Kationenaustauscherharz ist.

6. Verfahren nach Anspruch 5, dadurch gekennzeichnet, daß das Kationenaustauscherharz in der Natriumform vorliegt.

7. Verfahren nach Anspruch 1, dadurch gekennzeichnet, daß das Harz ein nicht-funktionelles Copolymer von Divinylbenzol und Styrol, ein Ionenaustauscherharz, das ein Copolymer von Divinylbenzol und Styrol ist, oder ein Gemisch dieser Harze ist.

8. Verfahren nach einem der vorhergehenden Ansprüche, dadurch gekennzeichnet, daß die Probe der Körperflüssigkeit Blut, Urin, Spinalflüssigkeit oder Peritonealflüssigkeit ist.

9. Nährmedium zur Bestimmung biologischer Aktivität in einer Probe einer Körperflüssigkeit, die antimikrobielles Material enthalten kann, wobei das Medium eine wäßrige Dispersion biologischer Nährstoffe sowei eine wirksame Menge eines Harzes umfaßt, das zur Isolierung antimikrobieller Materialien fähig ist, ohne die biologische Aktivität während des Kultivierens der Probe einer Körperflüssigkeit zu stören, wobei das Harz ein Ionenaustauscherharz und/oder ein nicht-funktionelles Adsorberharz ist.

10. Nährmedium nach Anspruch 9, dadurch gekennzeichnet, daß das Harz ein Ionenaustauscherharz ist.

11. Nährmedium nach Anspruch 9, dadurch gekennzeichnet, daß das Harz ein nicht-funktionelles Adsorberharz ist.

12. Nährmedium nach Anspruch 9, dadurch gekennzeichnet, daß das Harz ein nicht-funktionelles Adsorberharz zusammen mit einem Ionenaustauscherharz ist.

13. Nährmedium nach Anspruch 10 oder 12, dadurch gekennzeichnet, daß das Ionenaustauscherharz ein Kationenaustauscherharz ist.

14. Nährmedium nach Anspruch 13, dadurch gekennzeichnet, daß das Kationenaustauscherharz in der Natriumform vorliegt.

15. Nährmedium nach Anspruch 9, dadurch gekennzeichnet, daß das Harz ein nicht-funktionelles Copolymer von Divinylbenzol und Styrol, ein Ionenaustauscherharz, das ein Copolymer von Divinylbenzol und Styrol ist, oder ein Gemisch dieser Harze ist.

**Revendications**

1. Procédé pour détecter l'activité biologique dans un échantillon de fluide corporel qui peut contenir une matière antimicrobienne, où un milieu de croissance est inoculé avec l'échantillon de fluide corporel, le milieu de croissance inoculé est cultivé et les sousproduits métaboliques sont surveillés pour détecter l'activité biologique, caractérisé en ce qu'une résine capable d'isoler n'importe quelle matière antimicrobienne présente dans l'échantillon de fluide corporel sans gêner l'activité biologique durant la période de culture est incorporée dans ce milieu de croissance, la résine étant une résine échangeuse d'ions et/ou un adsorbant résineux non fonctionnel.

2. Procédé selon la revendication 1, dans lequel la résine est une résine échangeuse d'ions.

3. Procédé selon la revendication 1, dans lequel la résine est un adsorbant résineux non fonctionnel.

4. Procédé selon la revendication 1, dans lequel la résine est un adsorbant résineux non fonctionnel avec une résine échangeuse d'ions.

5. Procédé selon la revendication 2 ou la revendication 4, dans lequel la résine échangeuse d'ions est une résine échangeuse de cations.

6. Procédé selon la revendication 5, dans lequel la résine échangeuse de cations est sous la forme sodium.

7. Procédé selon la revendication 1, dans lequel la résine est un copolymère non fonctionnel de divinylbenzène et de styrène, une résine échangeuse d'ions qui est un copolymère de divinylbenzène et de styrène, ou leur mélange.

8. Procédé selon l'une quelconque des revendications précédentes, dans lequel l'échantillon de fluide corporel est du sang, de l'urine, un fluide spinal ou un fluide du péritoine.

9. Milieu de croissance pour détecter l'activité biologique dans un échantillon de fluide corporel qui peut contenir une matière antimicrobienne, le milieu comprenant une dispersion aqueuse de matières nutritives biologiques et une quantité efficace d'une résine capable d'isoler des matières antimicrobiennes sans gêner l'activité biologique durant la culture d'un échantillon de fluide corporel, la résine étant une résine échangeuse d'ions et/ou un adsorbant résineux non fonctionnel.

10. Milieu de croissance selon la revendication 9, dans lequel la résine est une résine échangeuse d'ions.

11. Milieu de croissance selon la revendication 9, dans lequel la résine est un adsorbant résineux non fonctionnel.

12. Milieu de croissance selon la revendication 9, dans lequel la résine est un adsorbant résineux non fonctionnel avec une résine échangeuse d'ions.

13. Milieu de croissance selon la revendication 10 ou la revendication 12, dans lequel la résine échangeuse d'ions est une résine échangeuse le cations.

14. Milieu de croissance selon la revendication 13, dans lequel la résine échangeuse de cations est sous la forme sodium.

15. Milieu de croissance selon la revendication 9, dans lequel la résine est un copolymère non fonctionnel de divinylbenzène et de styrène, une résine échangeuse d'ions qui est un copolymère de divinylbenzène et de styrène, ou leur mélange.